(19) Europäisches Patentamt
European Patent Office
Office européen des brevets

(11) **EP 2 055 197 B1**

(12) **FASCICULE DE BREVET EUROPEEN**

(45) Date de publication et mention de la délivrance du brevet:
**20.05.2015 Bulletin 2015/21**

(51) Int Cl.:
***A23L 1/236*** *(2006.01)* ***C07H 15/04*** *(2006.01)*

(21) Numéro de dépôt: **08167680.1**

(22) Date de dépôt: **27.10.2008**

(54) **Procédé d'évapocristallisation du maltitol**

Verfahren zum Verdampfen und Kristallisieren von Maltitol

Method for evaporative crystallisation of maltitol

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **30.10.2007 FR 0758704**

(43) Date de publication de la demande:
**06.05.2009 Bulletin 2009/19**

(73) Titulaire: **Roquette Frères**
**62136 Lestrem (FR)**

(72) Inventeurs:
• **Barata, Manuel**
**62920 GONNEHEM (FR)**
• **Duflot, Pierrick**
**62136 LA COUTURE (FR)**
• **Gharsallaoui, Adem**
**21000 DIJON (FR)**
• **Mathlouthi, Mohamed**
**51390 COULOMMES-LA-MONTAGNE (FR)**

(74) Mandataire: **Cabinet Plasseraud**
**52, rue de la Victoire**
**75440 Paris Cedex 09 (FR)**

(56) Documents cités:
**EP-A- 0 139 573 EP-A- 0 189 704**
**WO-A-02/04473 US-A- 4 408 041**

• **SCHOUTEN A ET AL: "A redetermination of the crystal and molecular structure of maltitol (4-O-alpha-d-glucopyranosyl-d-glucitol)" CARBOHYDRATE RESEARCH, ELSEVIER SCIENTIFIC PUBLISHING COMPANY. AMSTERDAM, NL, vol. 322, no. 3-4, 12 décembre 1999 (1999-12-12), pages 298-302, XP004184154 ISSN: 0008-6215**

**Description**

L'invention concerne un procédé de cristallisation du maltitol caractérisé en ce que la cristallisation est conduite par évaporation sous vide en un temps très court.

L'invention est plus particulièrement relative à un procédé consistant à évaporer une solution de maltitol à haute richesse en maltitol, initialement sous-saturée, de manière à l'amener à sursaturation dans la zone métastable du maltitol à la température mesurée sous vide partiel.

L'ensemencement est alors réalisé avec des semences cristallines de maltitol dont la taille et la dispersion dans la solution sursaturée sont contrôlées.

L'invention consiste ensuite à maintenir constant l'état de sursaturation en maltitol pendant toute la durée de la croissance des cristaux par le contrôle des conditions de température, d'agitation par pompage des buées en cours d'évaporation et d'alimentation en sirop de maltitol à cristalliser.

L'invention permet alors d'obtenir en moins de 2 heures des cristaux de maltitol dont la répartition granulométrique est parfaitement contrôlée.

Le maltitol (1,4-O-$\alpha$-D-glucopyranosyl-D-glucitol) est un polyol obtenu par hydrogénation du maltose.

L'absence d'extrémité réductrice dans la molécule de maltitol lui confère une stabilité élevée tant sur le plan thermique que sur le plan chimique.

Moins calorique que le saccharose, il possède des propriétés organoleptiques voisines de ce sucre. Non cariogène, il est alors utilisé dans bon nombre d'applications tant alimentaires que pharmaceutiques.

La seule forme cristalline connue à ce jour pour le maltitol est la forme anhydre, décrite dans le brevet US 4.408.041.

Ce n'est en effet qu'en 1983, date de publication de ce brevet, que la société HAYASHIBARA décrivait pour la première fois la production de cristaux de maltitol.

Auparavant, ce polyol avait toujours été considéré comme un produit non cristallisable. Ce postulat erroné trouve en réalité son origine dans le fait que la cristallisation du maltitol à partir d'une solution sursaturée n'est pas aussi facile à contrôler que dans le cas d'autres polyols comme le mannitol ou l'érythritol par exemple. Par ailleurs, les solutions de maltitol contenaient bon nombre d'impuretés constituées de polyols de degré de polymérisation supérieur ou égal à 3.

Certaines caractéristiques propres au maltitol, comme en particulier sa solubilité, sont également à l'origine des difficultés constatées.

Au sens de l'invention, on entend par « solubilité du maltitol » la concentration en maltitol d'une solution saturée qui est en équilibre avec le maltitol à l'état solide.

La « sursaturation en maltitol » d'une solution de maltitol, à une température donnée, se définit au sens de l'invention comme le rapport de la masse du maltitol sur la masse d'eau de la solution, ramenée à la masse de maltitol sur la masse d'eau de la solution saturée à l'état pur.

La courbe de solubilité du maltitol ainsi que la courbe de limite du degré de sursaturation (notée $\sigma$) en fonction de la température traduisent classiquement le comportement du maltitol en solution et partagent le domaine des concentrations en fonction de la température en trois zones :

1) en dessous de la saturation ($\sigma$ < 1) où la cristallisation n'est pas possible,
2) entre $\sigma$ = 1 et $\sigma$ = 1,08, zone métastable où nucléation et croissance peuvent se produire, et
3) au dessus de $\sigma$ = 1,08, zone labile où nucléation spontanée, croissance et amorphisation peuvent avoir lieu de manière non contrôlée.

La zone métastable d'un composé donné est classiquement caractérisée par la naissance spontanée de germes cristallins qui peuvent croître ou disparaître, les germes s'y formant ainsi sont non uniformes et de géométries irrégulières.

Pour le maltitol, la limite de sursaturation de la zone métastable est $\sigma$ = 1,08 (SCHOUTEN et al. 1999, Carbohydrate Research, 322, 298-302). Cette valeur est relativement faible par rapport à celle des autres sucres, et rend le contrôle de la cristallisation particulièrement délicate.

La solubilité du maltitol dans l'eau est d'une importance fondamentale pour la détermination de la sursaturation qui représente la force motrice régissant la croissance du cristal.

Parmi tous les facteurs susceptibles d'influencer la solubilité du maltitol dans l'eau, la température est considérée comme le facteur présentant l'effet le plus remarquable (OHNO et HIRAO, 1982, Carbohydrate Research, 108, 163-171).

La solubilité du maltitol croit en effet significativement avec la température. A titre indicatif, sa solubilité passe de 132,5 g pour 100 ml d'eau à 8,5°C, à 567,3 g pour 100 ml d'eau à 90°C (*article de* A. GHARSALLAOUI et al. sous presse dans Food Biophysics).

La mise en oeuvre de ces règles de base dans les procédés de cristallisation du maltitol décrits dans l'état de la technique n'a donné lieu jusqu'à présent qu'à des succès mitigés.

En effet, la cristallisation est une opération unitaire complexe faisant intervenir de nombreux facteurs pas toujours connus et maîtrisés.

Il faut également signaler que la forme finale d'un cristal dépend largement des conditions de cristallisation. Les impuretés peuvent également modifier la forme (ou habitus) du cristal, telles les formes prismatiques et bipyramidales du cristal de maltitol en présence de différentes concentrations de maltotriitol, décrites par la société Demanderesse dans son brevet EP 905.138.

Les impuretés ont également bien d'autres incidences sur la cristallisation, jusqu'à l'empêcher complètement.

D'autre part, si la cristallisation n'est pas maîtrisée, on peut même obtenir un produit amorphe dont la structure peut évoluer vers un état plus stable. Cette « transition vitreuse » dépend de la structure et des conditions environnementales de température et d'humidité.

En général, la cristallisation est considérée comme un processus physique qui met systématiquement en oeuvre deux mécanismes de base : la nucléation et la croissance des cristaux.

Les deux phénomènes se produisent dans la solution sursaturée, et, au regard du mode d'obtention de la solution sursaturée, on distingue différents modes de cristallisation : les procédés thermiques dans lesquels la sursaturation est réalisée par refroidissement, évaporation ou combinaison des deux, et les procédés avec ajout d'un co-solvant ou d'un additif modifiant les propriétés du système.

Lorsque la solubilité du composé à cristalliser augmente rapidement avec la température, le refroidissement peut constituer le procédé approprié pour cristalliser, et c'est souvent en effet le procédé le plus utilisé.

La solution est refroidie jusqu'à un certain niveau de sursaturation. La création d'interfaces (ou germes cristallins) peut être obtenue par un refroidissement brusque ou lent, ou dans la majorité des cas, par un ensemencement.

Le choix dépend en fait de la nature du soluté et des cristaux désirés.

Le grossissement des cristaux s'opère généralement par la diminution graduelle de la température. Dans ce type de procédé, le profil de température est donc le facteur primordial de contrôle. L'écart de température entre la masse en cours de cristallisation et le fluide de refroidissement ne doit pas être trop élevé, au risque de voir se former sur la surface métallique de l'échangeur thermique servant au refroidissement un dépôt solide appelé « croute » ou « blindage », créant une résistance supplémentaire au transfert.

Industriellement, la cristallisation par refroidissement du maltitol n'est par ailleurs pas limitée par la vitesse de croissance des cristaux, mais par la capacité de refroidissement de la solution mise à cristalliser.

Dans le cas du maltitol, la phase de cristallisation par refroidissement est ainsi souvent précédée d'une concentration par évaporation, qui permet l'obtention puis le maintien de la sursaturation.

Il apparaît ainsi qu'un rendement acceptable ne peut être obtenu en une seule étape sous peine d'obtenir un produit de pureté réduite.

Dans le cas du maltitol, on opère généralement en plusieurs étapes, l'eau mère issue de la première cristallisation, après séparation des cristaux, servant à alimenter une nouvelle cristallisation.

Une alternative à cette étape de cristallisation par épuisement peut être le traitement de l'eau mère par chromatographie, généralement en lit mobile simulé.

Il peut être également procédé à la combinaison d'une étape d'évaporation et une étape de refroidissement en introduisant la solution chaude dans une enceinte à pression réduite.

Cela entraîne la vaporisation spontanée (« flash »), la réduction de pression progressive permettant de maintenir la sursaturation. A partir d'une certaine valeur de sursaturation, on procède au refroidissement.

Les premières poudres semi-cristallines de maltitol commercialisées ont été préparées par une technique dite de « massé », consistant à faire prendre en masse une solution déshydratée de maltitol présentant une richesse pouvant atteindre au mieux 90 % par ajout d'une semence composée de cristaux de sucres ou de polyols. Un tel procédé est par exemple décrit dans les brevets JP 57-47680 et JP 58-158145.

Il a été également proposé dans le brevet US 4.408.401, de préparer des mélanges cristallins pulvérulents, appelés « *total sugar* », par atomisation de solutions pré-cristallisées ou masses cuites. Celles-ci sont obtenues par refroidissement très lent d'une solution aqueuse sursaturée en maltitol, contenant par ailleurs de larges quantités de polyols tels que le sorbitol, maltotriitol, maltotétraitol et d'autres polyols de degré de polymérisation supérieur.

Ce refroidissement très lent, et cet ajout d'une semence cristalline de maltitol sont en effet, dans ce procédé, nécessaires à l'apparition et à la croissance des cristaux de maltitol.

Cependant, ce « *total sugar* » est loin d'être suffisamment cristallin, puisqu'il nécessite également d'être séché davantage, pendant 40 minutes environ, mais aussi d'être maturé pendant 10 heures.

Toutefois, sa stabilité à la vapeur d'eau est médiocre, et on déplore souvent sa prise en masse au stockage.

La société Demanderesse, par ses brevets EP 185.595 et EP 189.704 a contribué à apporter une première solution à ces difficultés en proposant la préparation de poudres cristallines de maltitol de très haute richesse, par la mise en oeuvre de procédés basés sur l'emploi de techniques de fractionnement par chromatographie continue.

Ces procédés permettent d'obtenir à coût compétitif des poudres d'une pureté supérieure à 99 %, par la cristallisation en solution aqueuse du maltitol présent dans la fraction chromatographique particulièrement riche en ce polyol.

Les techniques dites de « massé » d'une part et de cristallisation en solution aqueuse d'autre part, ont pendant longtemps été considérées comme les seuls procédés employés industriellement pour produire du maltitol cristallisé.

Cependant, sur le plan industriel, l'étape la plus difficile à maîtriser reste le contrôle de la taille et de la forme des cristaux.

La cristallisation du maltitol génère en effet des poussières par attrition dans les cristalliseurs de grande capacité, par refroidissement ou au cours du séchage.

La présence de fins cristaux, la non-homogénéité de la granulométrie et de l'aptitude naturelle à l'hygroscopicité du maltitol ont amené les spécialistes du domaine de la cristallisation des polyols à rechercher une méthode de cristallisation susceptible d'assurer une stabilité prolongée vis-à-vis de la vapeur d'eau et de la température dans les conditions habituellement pratiquées dans le stockage, le transport et le conditionnement du maltitol cristallisé en poudre.

A côté des techniques dites de « massé » et de cristallisation par refroidissement en solution aqueuse, les procédés de cristallisation par évaporation ont été alors travaillés.

Mais ces travaux visant à cristalliser le maltitol ont pour la plupart utilisé des procédés classiques trop longs, mêlant évaporation et refroidissement.

En effet, il s'est souvent agi d'assurer la maturation de cristaux dans un massé, repris pour être séché et broyé sans prise en compte des conséquences mécaniques comme la brisure des cristaux, la formation de poussières et l'augmentation de l'hygroscopicité. On peut citer par exemple :

- la demande de brevet US 2006/0078662 qui enseigne la fabrication de maltitol sans ensemencement. La solution est d'abord concentrée par évaporation jusqu'à une humidité résiduelle de 4,5 à 6 %, puis refroidie à une température de 10 à 20°C. La solution concentrée et refroidie alimente enfin une extrudeuse.
  Le produit obtenu après broyage présente une granulométrie trop dispersée, entre 50 et 500 $\mu$m, avec une humidité résiduelle de 0,2 à 0,8 %, caractéristique d'un produit instable, et un point de fusion compris entre 144 et 148°C, caractéristique d'un produit de faible pureté (le point de fusion du maltitol pur étant compris entre 146 et 147°C).
- la demande de brevet internationale WO 2005/014608 décrit la préparation d'un sirop riche en maltose (de 70 à 80 % sur sec) enrichi par séparation chromatographique (teneur en maltose de plus de 92 % sur sec), puis hydrogéné catalytiquement avant concentration puis solidification ou cristallisation et séchage. Le maltitol cristal présente une matière sèche de 98,5 % pour une richesse en maltitol supérieure ou égale à 97 %.
  Mais lorsque la cristallisation est utilisée pour purifier la solution de maltitol en sortie de chromatographie, c'est surtout par le biais de multiples étapes longues, lourdes et fastidieuses.
- Le brevet EP 139573 consiste en une évaporation « flash » de surface et un recyclage en continu de la solution de maltitol qui est réchauffée à l'extérieur de l'évaporateur dans un échangeur thermique utilisant les calories de la vapeur comprimée.
  On prélève une partie de la solution avec les cristaux pour séparer lesdits cristaux des eaux mères qui sont ensuite recyclées. On collecte les cristaux en partie par décantation dans une jambe du cristallisoir.
  Cependant, cette méthode comporte de multiples opérations unitaires (flash évaporation, compression de vapeur, échange de chaleur, décantation) qui ne la rend pas particulièrement attractive ni industriellement rentable.
- La demande de brevet internationale WO 02/04473 couvre enfin la préparation de cristaux de maltitol par un procédé d'évapocristallisation conduit dans des conditions de laboratoire, calqué sur les procédés de cristallisation classiquement mis en oeuvre en sucrerie.

Cette méthode consiste surtout en une évapocristallisation menée avec des impuretés ajoutées à un sirop de maltose hydrogéné contenant un spectre étendu d'oligomères résiduels.

Cette demande de brevet WO 02/4473 décrit l'obtention de maltitol cristallisé avec un rendement de 50 à 80 % en maltitol et de 60 à 70 % de matière sèche. Ces rendements sont obtenus après évapocristallisation, par la différence de matières sèches entre les eaux mères et les cristaux.

De telles concentrations de matières sèches ne peuvent être transposées en conditions industrielles, étant donné les difficultés qu'elles occasionneraient pour les étapes de pompage et de centrifugation.

Par ailleurs, la cristallisation par évaporation n'est pas utilisée seule ; il est nécessaire de la combiner avec une étape de refroidissement.

En fait, seule l'amorce de cristallisation est préparée par évaporation, la cristallisation proprement dite étant réalisée classiquement par refroidissement en 30 heures.

On constate surtout que les sursaturations pratiquées sont trop élevées pour pouvoir contrôler efficacement la taille ou la forme des cristaux obtenus.

Il faut en effet rappeler que la limite de la zone métastable du maltitol est $\sigma = 1,08$, et que les sursaturations mentionnées dans cette demande de brevet WO 02/4473 se situent dans la zone labile, qui par définition est incontrôlable.

Enfin, les rendements mal définis se rapportent plus à la masse de crème de maltitol retenue dans la centrifugeuse plutôt qu'à un vrai rendement en cristaux.

De tout ce qui précède, il résulte qu'il existe un besoin non satisfait de disposer d'un procédé de cristallisation du maltitol permettant de maîtriser la taille des cristaux de maltitol et leur forme, sans qu'il ne soit nécessaire de mettre en oeuvre des étapes longues, lourdes, difficiles et complexes.

La Société Demanderesse a eu le mérite de concilier tous ces objectifs réputés jusqu'alors difficilement conciliables en proposant un procédé d'évapocristallisation du maltitol remarquablement rapide, qui repose sur le contrôle de la qualité des semences de cristallisation et des conditions de solubilité du maltitol et de sursaturation de la solution de maltitol pendant toute la croissance des cristaux.

La présente invention est ainsi relative à un procédé de préparation de cristaux de maltitol par évapocristallisation, caractérisé par le fait qu'il consiste à :

a) préparer une solution de maltitol d'au moins 85 % de richesse en maltitol sur sec, de préférence comprise entre 89 % et 99 % de richesse en maltitol sur sec, plus préférentiellement encore comprise entre 93 et 95 % de richesse en maltitol sur sec,
b) concentrer sous vide ladite solution de maltitol dans l'évapocristallisoir de manière à obtenir un sirop de maltitol dont le degré de sursaturation en maltitol est situé dans la zone métastable du maltitol,
c) ensemencer la solution sursaturée en maltitol avec une semence cristalline de maltitol sous forme dispersée,
d) conduire la cristallisation en maintenant constant le degré de sursaturation en maltitol dans la zone métastable du maltitol par le contrôle des conditions de température, d'agitation forte par pompage des buées d'évaporation et de l'alimentation en sirop de maltitol à cristalliser,
e) récupérer les cristaux ainsi obtenus. Le procédé de cristallisation par évaporation selon l'invention consiste à évaporer la solution de maltitol jusqu'à sursaturation, puis à ensemencer et maintenir un degré de sursaturation constant afin de réaliser un grossissement des cristaux de semence.

La première étape consiste en la préparation d'une solution de maltitol d'au moins 85 % de richesse en maltitol sur sec, de préférence comprise entre 89 % et 99 % de richesse en maltitol sur sec, plus préférentiellement encore comprise entre 93 et 95 % de richesse en maltitol sur sec.

La notion de richesse doit être entendue dans la présente invention comme correspondant au pourcentage de maltitol exprimé en poids sec/sec par rapport à l'ensemble des carbohydrates présents dans la composition cristalline de maltitol.

Ces carbohydrates peuvent être des polyols tels qu'en particulier le sorbitol, les polyols de degré de polymérisation (ou DP) de 3 et les polyols de DP 4. Habituellement, cette richesse est mesurée par chromatographie liquide haute performance.

La préparation de la solution de maltitol s'effectue par toute méthode connue par ailleurs de l'homme du métier.

On peut par exemple suivre l'enseignement du brevet EP 905.138 dont la société Demanderesse est titulaire, en contrôlant les étapes des procédés décrits de manière à ce que le sirop de maltitol contienne au moins 85 % de richesse en maltitol sur sec, de préférence comprise entre 89 % et 99 % de richesse en maltitol sur sec, plus préférentiellement encore comprise entre 93 et 95 % de richesse en maltitol sur sec et une teneur en maltotriitol inférieure ou égale à 1% sur sec, de préférence inférieure ou égale à 0,5 % sur sec.

La deuxième étape du procédé conforme à l'invention consiste à concentrer sous vide la solution de maltitol ainsi préparée de manière à obtenir un sirop de maltitol dont le degré de sursaturation en maltitol est situé dans la zone métastable du maltitol.

La concentration de la solution de maltitol est réalisée par évaporation de l'eau de ladite solution, évaporation assurée par apport de vapeur saturée à travers un échangeur thermique placé soit dans l'évapocristallisoir, soit sur une boucle de recirculation.

Pour créer une sursaturation de la solution de maltitol ainsi obtenue, il faut agir sur la solution de manière à ce que la concentration en maltitol dépasse sa solubilité et conduise ainsi à un sirop initial présentant une sursaturation comprise entre 1,04 et 1,08, située dans la zone métastable du maltitol.

La société Demanderesse a déterminé que ces valeurs de sursaturation étaient atteintes, pour une solution de maltitol de richesse comprise entre 93 et 95 %, à une température de cuisson comprise entre 75 et 85°C et sous un vide partiel compris entre 20 et 30 kPa.

Comme il sera exemplifié ci-après, si la consigne est réglée à 22 kPa, cela correspond à une température de cuisson de 76°C.

La quantité d'eau à évaporer est déterminée par le calcul, à partir de la concentration initiale en maltitol.

La troisième étape du procédé conforme à l'invention consiste, après avoir atteint une valeur de sursaturation comprise entre 1,04 et 1,08, par exemple une valeur de 1.05 ou de 1,07, à ensemencer la solution sursaturée en maltitol avec une semence cristalline de maltitol sous forme dispersée.

Cet ensemencement est réalisé avec des cristaux de maltitol de dimension moyenne, mesurée par granulométrie laser, comprise entre 10 et 50 $\mu$m, de préférence comprise entre 20 et 30 $\mu$m, plus préférentiellement de l'ordre

de 25 μm.

Ces tailles sont déterminées sur un Granulomètre à diffraction LASER type LS 230 de la société BECKMAN-COULTER, équipé de son module de dispersion poudre (voie sèche), en suivant le manuel technique et les spécifications du constructeur.

La gamme de mesure du Granulomètre à diffraction LASER type LS 230 est de 0,04 μm à 2.000 μm.

Les conditions opératoires de vitesse de vis sous trémie et d'intensité de vibration de la goulotte de dispersion sont déterminées de manière à ce que la concentration optique soit comprise entre 4 % et 12 %, idéalement 8 %.

Les résultats sont calculés en % volumique, et exprimés en μm.

Les cristaux de maltitol utilisés comme semences sont dispersés dans une solution de maltitol saturée, ou dans du polyéthylène glycol, notamment du PEG 300, solvant alimentaire ayant une viscosité relativement élevée permettant la dispersion et la séparation des cristaux généralement agglomérés à cause de leur petite taille.

La dispersion des semences de cristallisation, grâce au vide partiel et à la température élevée, permet de minimiser l'agglomération et la formation de macles.

La quantité de semences introduite est calculée en fonction de la taille théorique des cristaux à obtenir selon la formule (f) suivante (d'après ROGE et MATHLOUTHI, dans AVH Association, 12th Symposium - Reims, March 2005) :

$$\frac{m_C}{m_S} = \left(\frac{L_C}{L_S}\right)^3$$

où :

nucléation spontanée), la phase de croissance des cristaux et
réalisée sous vide partiel maintenu constant.

L'évaporation est réalisée de telle manière à ce que l'on obtienne dans l'évapocristallisoir une agitation forte et bien contrôlée de la solution sursaturée de maltitol.

L'eau qui s'évapore de ladite solution va en effet créer un phénomène de pompage qui agite fortement la solution.

Ce phénomène permet d'agiter la solution avec une amplitude bien plus grande que celle qui serait générée par une agitation mécanique.

L'eau évaporée est alors soit éliminée de l'évapocristallisoir, ou condensée à l'intérieur de l'évapocristallisoir.

La matière sèche de la solution à l'entrée de l'évapocristallisoir est également ajustée de manière à obtenir la quantité de vapeur saturée nécessaire et suffisante au pompage.

Par ailleurs, il est à noter que l'agitation forte induite par ce contrôle de l'évaporation permet une homogénéité des transferts de chaleur et de matière qui aboutit, comme nous allons le démontrer, à une croissance des cristaux maîtrisée et à une réduction de la nucléation spontanée, génératrice de grains fins qui sont la cause des problèmes de prise en masse généralement constaté dans l'état de la technique.

La société Demanderesse recommande par ailleurs de réaliser cette quatrième étape du procédé conforme à l'invention par paliers successifs, chaque palier étant constitué soit :

- d'une phase de concentration par évaporation externe, puis,
- d'une phase de repos dite de condensation (évaporation interne), à concentration globale constante, comme il sera exemplifié ci-après.

Les phases de concentration par évaporation de la massecuite sont appelées la « montée de cuite ». Elles permettent de compenser la baisse de sursaturation de l'eau mère de maltitol induite par la croissance des cristaux.

L'évapocristallisation est conduite pendant une durée totale de moins de 2 heures, de préférence comprise entre 30 et 90 minutes, plus préférentiellement encore comprise entre 40 et 70 minutes.

A la connaissance de la Société demanderesse, il n'a jamais été décrit dans l'état de la technique de procédé de cristallisation du maltitol aussi rapide. Le temps de cristallisation est ainsi de 10 à 40 fois plus court que le temps nécessaire à la cristallisation par refroidissement.

La température de cuisson est quant à elle maintenue constante entre 70 et 85 °C, de préférence entre 72 et 80°C.

La cinquième et dernière étape du procédé conforme à l'invention consiste à récupérer le maltitol cristallisé.

Le lavage des cristaux est réalisé par toute méthode connue par ailleurs de l'homme du métier.

Les cristaux obtenus sont analysés en forme, taille et répartition granulométrique.

L'habitus du cristal est déterminé à la fois par microscopies optique et électronique.

Pour la microscopie optique, les cristaux sont déposés dans une coupelle en verre et examinés sous un binoculaire NIKON muni d'un objectif ayant un grossissement maximal de 250. Une caméra est reliée à un système d'acquisition d'images sur Ordinateur.

Les images sont acquises sur 256 niveaux de gris, et ont une résolution de 752x548 pixels. L'éclairage de l'échantillon est assuré par des fibres optiques fournissant une lumière froide favorable au maintien de l'échantillon dans son état initial.

Pour la microscopie électronique, les observations sont réalisées à l'aide d'un microscope électronique à balayage FEI type QUANTA 200 FEG.

Les cristaux sont observés sous une tension de 2 à 5 kV. Les photographies sont captées sur le microscope avec un grossissement de 50 à 350 fois puis agrandies lors de l'impression.

Les cristaux obtenus par cristallisation d'une solution de maltitol renfermant moins de 1 % en maltotriitol présentent une forme bipyramidale, comme la société Demanderesse l'enseigne dans son brevet EP 905.138.

La taille des cristaux, déterminée par Granulomètre laser, est comprise entre 50 et 550 $\mu$m, de préférence comprise entre 150 et 400 $\mu$m, plus préférentiellement comprise entre 150 et 350 $\mu$m.

La société Demanderesse a constaté que, de manière remarquable, la répartition granulométrique des cristaux de maltitol obtenus est de type Gaussienne, avec un coefficient de variation n'excédant pas 60 %, de préférence n'excédant pas une valeur comprise entre 45 et 55 %.

Il est à noter également que le procédé d'évapocristallisation conforme à l'invention, appliqué à des solutions de maltitol de 93 %, présentant de l'ordre de 0,5 % de maltotriitol, conduit rapidement à des cristaux de formes bipyramidales dont la distribution granulométrique très serrée permet d'obtenir une poudre de maltitol fluide, d'excellente coulabilité, et qui ne pose aucune problème de prise en masse dans des conditions de conservation variant dans de larges intervalles de température (de 15 à 30°C) et d'humidité relative (45 à 75 %).

Le rendement de cristallisation est enfin relativement élevé, compris entre 55 et 65 %, exprimé en poids sec des cristaux récupérés dans la massecuite sur le poids sec de ladite massecuite.

L'invention sera mieux comprise à la lecture de l'exemple qui suit et qui se veut illustratif et non limitatif.

**Exemple**

La cristallisation consiste à évaporer une solution de maltitol jusqu'à une concentration en maltitol située dans les limites de la zone métastable, puis à ensemencer et maintenir une sursaturation dans lesdites limites afin de réaliser un grossissement des cristaux de semences.

La cristallisation est réalisée dans un évapocristallisoir industriel muni d'un agitateur disposé dans un puits central, constituée d'un récipient de volume de 6 m$^3$. Un échangeur de chaleur tubulaire, de forme cylindrique, est alimenté par de la vapeur d'eau saturée. La vapeur est fournie grâce à une chaudière, à la pression de 1 bar (100 kPa). La température de cuisson est contrôlée en continu.

Le vide partiel est obtenu à l'aide d'une pompe à anneau liquide munie d'un manostat à membrane. La consigne est maintenue à 22 kPa ce qui correspond à une température de buée comprise entre 72 et 74°C et une température de cuisson de 76°C.

La montée de la cuite s'effectue par évaporation vers l'extérieur des buées, jusqu'à atteindre la matière sèche de sursaturation.

On alterne alors les phases d'évaporation de l'eau avec les phases de condensation interne de l'eau afin de maîtriser la sursaturation et surtout la forte agitation par pompage des buées pendant la cristallisation.

Un supplément de vapeur saturée vive est alimentée si nécessaire pour maintenir l'agitation de la massecuite.

La température en fin de cuite est de 80°C. Le grainage est assuré par l'introduction dans la cuite de cristaux de maltitol calibrés (de l'ordre de 25 $\mu$m) et dispersés dans du PEG 300 en quantité suffisante pour empêcher la décantation des semences.

La cristallisation s'effectue sous vide partiel (22 kPa) et se décompose comme suit :

- concentration de la solution par évaporation afin d'obtenir une sursaturation égale à 1,07. La quantité d'eau à évaporer est déterminée par le calcul, à partir de la concentration initiale.
- lorsque la sursaturation désirée est atteinte, ensemencement par l'introduction d'une quantité de semences calibrées, quantité calculée selon la formule (f) évoquée ci-dessus.

La période qui suit ce grainage est composée de plusieurs phases d'évaporation entrecoupées de périodes de repos, comme indiqué dans les tableaux ci-dessous.

La cristallisation par paliers dure 54 minutes, et la température est comprise entre 76 à 80°C. La récupération des cristaux est ensuite effectuée. Deux lavages consécutifs avec de l'eau permettent de récupérer une poudre de cristaux secs et fluides après centrifugation et séchage des cristaux.

Tableau I : composition de la solution de maltitol à cristalliser et conditions de cristallisation.

| | |
|---|---|
| Masse de la solution de maltitol | 2.000 kg |
| Concentration du sirop de maltitol | 58 % |
| Richesse en maltitol | 93 % sur sec |
| Teneur en maltotriitol | 0,5 % sur sec |
| Durée de l'étape initiale de concentration | 18 min |
| Matière sèche à la Tp d'ensemencement | 80 % |
| Masse des semences | 500 g dans du PEG 300 |
| Tp d'ensemencement | 76°C |
| Taille moyenne des semences | 25 $\mu$m |
| Sursaturation d'ensemencement | 1,07 |
| Pression | 22 kPa |
| Nbre de paliers d'évaporation | 3 |
| Nbre de paliers de condensation interne | 3 |
| Durée totale des paliers | 54 min |
| Masse d'eau évaporée | 700 kg |
| Masse d'eau condensée | 875 kg |
| Quantité de pompage par vapeur | ~10.000 m$^3$/h |
| Quantité de pompage de l'agitation mécanique | ~2.000 m$^3$/h |
| Tp d'arrêt de cristallisation | 80°C |

Tableau II : détail des paliers d'évaporation

| Paliers successifs de | Essai n°1 |
|---|---|
| Evaporation/condensation | 10 min |
| Condensation | 5 min |
| Evaporation/condensation | 8 min |
| Condensation | 10 min |
| Evaporation/condensation | 6 min |
| Condensation | 15 min |

Les paliers de condensation permettent de vaincre le ralentissement de la vitesse de cristallisation qui accompagne habituellement la diminution de la richesse en maltitol de la solution lors de la cristallisation.

Tableau III : caractérisation des cristaux obtenus

| | Essai n°1 |
|---|---|
| Masse des cristaux obtenus dans l'évapocristallisoir | 600 kg |
| Rendement | 51,7 % MS |
| Taille moyenne | 300 $\mu$m |
| Répartition granulométrique (coefficient de variation) | 52 % |
| Forme des cristaux | Bipyramidaux |

L'évaporation sous vide par le procédé conforme à l'invention permet de baisser considérablement le temps de cristallisation par rapport au procédé classique par refroidissement, et d'obtenir des cristaux bipyramidaux de taille homogène et de distribution granulométrique serrée.

## Revendications

1. Procédé de préparation de cristaux de maltitol par évapocristallisation, **caractérisé par le fait qu'**il consiste à

a) préparer une solution de maltitol d'au moins 85 % de richesse en maltitol sur sec, de préférence comprise entre 89 % et 99 % de richesse en maltitol sur sec, plus préférentiellement encore comprise entre 93 et 95 % de richesse en maltitol sur sec,
b) concentrer sous vide partiel compris entre 20 et 30 kPa ladite solution de maltitol, de richesse comprise entre 93 et 95 %, à une température de cuisson comprise entre 75 et 85°C, dans l'évapocristallisoir de manière à obtenir un sirop de maltitol dont le degré de sursaturation en maltitol est situé dans la zone métastable du maltitol,
c) ensemencer la solution sursaturée en maltitol avec une semence cristalline de maltitol sous forme dispersée,
d) conduire la cristallisation pendant une durée totale de moins de 2 heures, en maintenant constant le degré de sursaturation en maltitol dans la zone métastable du maltitol par le contrôle de la température entre 70 et 85°C, d'agitation forte par pompage des buées d'évaporation, par paliers successifs constitués d'une phase de concentration par évaporation externe, puis d'une phase de repos dite de condensation (évaporation interne), à concentration globale constante, et de l'alimentation en sirop de maltitol à cristalliser,
e) récupérer les cristaux ainsi obtenus.

2. Procédé selon la revendication 1, **caractérisé en ce que** l'ensemencement est réalisé avec des cristaux de taille comprise entre 10 et 50 $\mu$m, de préférence comprise entre 20 et 30 $\mu$m, plus préférentiellement de l'ordre de 25 $\mu$m.

3. Procédé selon l'une quelconque des revendications 1 à 2, **caractérisé en ce que** les semences cristallines de maltitol sont dispersées dans un solvant alimentaire de viscosité relativement élevée choisi dans le groupe constitué par du sirop de maltitol saturé et par du polyéthylène glycol, notamment du PEG 300, de manière à empêcher la formation d'agrégats ou de macles.

4. Procédé selon l'une quelconque des revendications 1 à 3, **caractérisé en ce que** l'évapocristallisation est conduite par paliers d'évaporation/condensation et de condensation pendant une durée comprise entre 30 et 90 minutes, de préférence comprise entre 40 et 70 minutes.

5. Procédé selon l'une quelconque des revendications 1 à 4, **caractérisées en ce que** les cristaux de maltitol obtenus présentent une granulométrie comprise entre 50 et 550 $\mu$m, de préférence comprise entre 150 et 400 $\mu$m, plus préférentiellement comprise entre 150 et 350 $\mu$m.

6. Procédé selon la revendication 5, **caractérisée en ce que** la répartition granulométrique des cristaux de maltitol obtenus est de type Gaussienne, avec un coefficient de variation n'excédant pas 60 %, de préférence n'excédant pas une valeur comprise entre 45 et 55 %.

## Patentansprüche

1. Verfahren zur Herstellung von Maltit-Kristallen durch Verdampfungskristallisation, **dadurch gekennzeichnet, dass** es besteht aus

a) Herstellen einer Maltit-Lösung von mindestens 85% Maltit-Gehalt bezogen auf Trockenmasse, vorzugsweise zwischen 89% und 99% Maltit-Gehalt bezogen auf Trockenmasse, mehr bevorzugt zwischen 93 und 95% Maltit-Gehalt bezogen auf Trockenmasse,
b) Aufkonzentrieren der Maltit-Lösung mit einem Gehalt zwischen 93 und 95%, unter teilweisem Vakuum zwischen 20 und 30 kPa, bei einer Kochtemperatur zwischen 75 und 85 °C, in dem Verdampfungskristallisator, um einen Maltitsirup zu erhalten, dessen Maltit-Übersättigungsgrad in dem metastabilen Bereich des Maltits liegt,
c) Impfen der mit Maltit übersättigten Lösung mit einem Maltit-Impfkristall in dispergierter Form,
d) Durchführen der Kristallisation während eines gesamten Zeitraums von weniger als 2 Stunden, durch Konstanthalten des Maltit-Übersättigungsgrads in dem metastabilen Bereich des Maltits durch die Steuerung/Regelung der Temperatur zwischen 70 und 85 °C, des Grads des kräftigen Rührens durch Pumpen der evaporierten

**EP 2 055 197 B1**

Dämpfe, in aufeinander folgenden Stufen bestehend aus einer Konzentrierungsphase durch externe Verdampfung, dann einer Ruhephase, genannt Kondensationsphase (interne Verdampfung), bei konstanter globaler Konzentration, und des Grads des Zuführens des zu kristallisierenden Maltitsirups,
e) Gewinnen der so erhaltenen Kristalle.

**2.** Verfahren nach Anspruch 1, **dadurch gekennzeichnet, dass** das Impfen mit Kristallen durchgeführt wird, welche eine Größe zwischen 10 und 50$\mu$ m, vorzugsweise zwischen 20 und 30 $\mu$m, mehr bevorzugt von etwa 25 $\mu$m aufweisen.

**3.** Verfahren nach einem der Ansprüche 1 bis 2, **dadurch gekennzeichnet, dass** die Maltit-Impfkristalle in einem Nährlösungsmittel dispergiert sind, welches eine relativ hohe Viskosität aufweist, ausgewählt aus der Gruppe bestehend aus gesättigtem Maltitsirup und Polyethylenglykol, insbesondere PEG 300, um die Bildung von Aggregaten oder Kristallzwillingen zu verhindern.

**4.** Verfahren nach einem der Ansprüche 1 bis 3, **dadurch gekennzeichnet, dass** die Verdampfungskristallisation in Stufen von Verdampfung/Kondensation und Kondensation über einen Zeitraum zwischen 30 und 90 Minuten, vorzugsweise zwischen 40 und 70 Minuten durchgeführt wird.

**5.** Verfahren nach einem der Ansprüche 1 bis 4, **dadurch gekennzeichnet, dass** die erhaltenen Maltit-Kristalle eine Korngröße zwischen 50 und 550 $\mu$m, vorzugsweise zwischen 150 und 400 $\mu$m, mehr bevorzugt zwischen 150 und 350 $\mu$m aufweisen.

**6.** Verfahren nach Anspruch 5, **dadurch gekennzeichnet, dass** die Korngrößenverteilung der erhaltenen Maltit-Kristalle vom Gauß-Typ ist, mit einem Variationskoeffizient von nicht mehr als 60%, vorzugsweise der einen Wert von zwischen 45 und 55% nicht übersteigt.

**Claims**

**1.** Process for preparing maltitol crystals by evapocrystallization, wherein it consists in:

a) preparing a maltitol solution with a maltitol richness of at least 85% based on dry matter, preferably a maltitol richness of between 89% and 99% based on dry matter, and more preferably a maltitol richness of between 93% and 95% based on dry matter,
b) concentrating under a partial vacuum of between 20 and 30 kPa said maltitol solution with a maltitol richness of between 93% and 95% at a cooking temperature of between 75 and 85°C in an evapocrystallizer so as to obtain a maltitol syrup whose degree of maltitol supersaturation is within the metastable zone of maltitol,
c) seeding the supersaturated maltitol solution with a maltitol seed crystal in dispersed form,
d) performing the crystallization for a duration of less than 2 hours while keeping constant the degree of maltitol supersaturation within the metastable zone of maltitol by controlling the conditions of temperature between 70 to 85°C, conditions of vigorous stirring by pumping the evaporation vapors, by successive stages, each stage being constituted of a concentration phase by external evaporation, and then of a "condensation" resting phase (internal evaporation), at a constant overall concentration, of evaporation/condensation and of condensation and conditions of feeding in of the maltitol syrup to be crystallized,
e) recovering the crystals thus obtained.

**2.** Process according to Claim 1, wherein the seeding is performed with crystals between 10 and 50 $\mu$m in size, preferably between 20 and 30 $\mu$m and more preferably of about 25 $\mu$m in size.

**3.** Process according to Claim 1 or 2, **characterized in that** the maltitol seed crystals are dispersed in a food-grade solvent of relatively high viscosity chosen from the group consisting of saturated maltitol syrup and polyethylene glycol, especially PEG-300, so as to prevent the formation of aggregates or twins.

**4.** Process according to any one of Claims 1 to 3, wherein the evapocrystallization is performed by stages of evaporation/condensation and of condensation for a duration between 30 and 90 minutes, preferably between 40 and 70 minutes.

**5.** Process according to any one of Claims 1 to 4, wherein the maltitol crystals obtained have a particle size of between

50 and 550 $\mu$m, preferably between 150 and 400 $\mu$m and more preferably between 150 and 350 $\mu$m.

6. Process according to Claim 5, wherein the particle size distribution of the maltitol crystals obtained is of Gaussian type, with a coefficient of variation not exceeding 60%, and preferably not exceeding a value of between 45% and 55%.

## RÉFÉRENCES CITÉES DANS LA DESCRIPTION

**Documents brevets cités dans la description**

- US 4408041 A
- EP 905138 A
- JP 57047680 A
- JP 58158145 A
- US 4408401 A
- EP 185595 A
- EP 189704 A
- US 20060078662 A
- WO 2005014608 A
- EP 139573 A
- WO 0204473 A
- WO 024473 A

**Littérature non-brevet citée dans la description**

- **SCHOUTEN et al.** *Carbohydrate Research,* 1999, vol. 322, 298-302
- **OHNO ; HIRAO.** *Carbohydrate Research,* 1982, vol. 108, 163-171
- **A. GHARSALLAOUI et al.** *Food Biophysics*
- **ROGE ; MATHLOUTHI.** *12th Symposium - Reims,* Mars 2005